# EUROPEAN PATENT APPLICATION

(11) **EP 4 268 961 A1**
(43) Date of publication of application: **01.11.2023**
(21) Application number: 22170909.0
(22) Date of filing: 29.04.2022
(51) Int. Cl.: B01L 7/00, B01L 9/00, C12Q 1/68

(54) **SYSTEM FOR NUCLEIC ACID ANALYSIS**

(71) Applicant: miDiagnostics NV, 3001 Heverlee (BE)
(72) Inventor: Pauwelyn, Thomas, 3001 Heverlee (BE); Martin, Simone, 3001 Heverlee (BE); Willshare, Thomas, 3001 Heverlee (BE); Zandman, Jonathan, 7521 Enschede (NL); Ansink, Jos, 7521 Enschede (NL)
(74) Representative: AWA Sweden AB

(57) **Abstract**

With the present inventive concept there is provided a system comprising: a test card receiver configured to receive a test card, wherein the test card comprises a sample portion having a first side and a second side opposite the first side, wherein the sample portion of the test card is configured to hold a sample for PCR; a temperature adjuster configured to adjust a temperature of the sample portion of the test card over a plurality of temperature cycles, wherein each temperature cycle is between a first temperature and a second temperature different than the first temperature, wherein the temperature adjuster comprises a surface configured to be in contact with the first side of the sample portion of the test card; wherein the test card receiver comprises a spring member configured to push the sample portion of the test card towards the surface of the temperature adjuster; and a monitoring arrangement configured to monitor the sample portion of the test card over the plurality of temperature cycles, the monitoring arrangement comprising: at least one light sensor; an illumination system configured to illuminate the second side of the sample portion with light having wavelengths within at least three excitation wavelengths ranges; an emission filter configured to transmit light having wavelengths within at least three emission wavelength ranges; and collection optics configured to collect light emanating from the second side of the sample portion of the test card and transmitted through the emission filter, and wherein the collection optics is further configured to direct the collected light towards the light sensor such that at least a portion of the collected light impinges on the light sensor, wherein the light sensor is configured to generate at least one signal, wherein each signal pertains to light impinging on the light sensor after a unique number of temperature cycles of the plurality of temperature cycles.

## Description

### Technical field

The present inventive concept relates to a system for nucleic acid analysis.

### Background of the invention

A nucleic acid test is a technique used to detect a particular nucleic acid sequence analyte and thus usually to detect and identify a particular species or subspecies of organism, often a virus or bacterium that acts as a pathogen in a sample such as blood, tissue or urine. The amount of a certain genetic DNA or RNA analyte may be very small, and many nucleic acid tests include a step that amplifies the genetic analyte.

Although several ways of amplification exist, real time polymerase chain reaction is the best-established method for the detection and typing of different nucleic acid analytes in the areas of clinical diagnostics. Using PCR, single or multiple copies of sequences of DNA or RNA may be amplified into many, such as, thousands or millions of copies. The high number of copies generated with PCR makes PCR useful in qualitative and quantitative analysis of presence of nucleic acid analyte(s) in a sample.

There are two strategies for the visualization of amplified nucleic acid fragments; non-specific fluorescent dyes and fluorescently labeled oligonucleotide probes. The exponential production of PCR products during amplification causes a concomitant exponential increase of fluorescence. In multiplex PCR settings, more than one target biomarker sequence within the same sample can be amplified in the qPCR reaction and different fluorescently labelled sequence-specific oligonucleotides, e.g. probes or primers, allow for simultaneous or sequential target detection. Detection of the fluorescent signal can be done at any phase of the PCR reaction, e.g. at the final PCR phase (endpoint PCR) or in real-time during the exponential phase of the amplification reaction.

Real-time reverse transcription polymerase chain reaction (RT-PCR) is a variation of PCR and allows the use of RNA as template, The RNA template is first converted into complementary (c)DNA, which serves later as a template for exponential amplification using PCR. Coronavirus disease 2019 (COVID-19) can be tested via RT-PCR amplification and detection of the Coronavirus SARS-CoV-2 in a sample from the test person, such a s a sample obtained by, for example, nasopharyngeal or throat swabbing.

Rapid screening and low-cost diagnosis play a crucial role in choosing the correct course of intervention and in controlling disease outbreaks. This is especially important if the disease has no effective treatment, as is for instance the case for COVID-19 infections. Infection control and prevention in such cases especially depends on disrupting the transmission of pathogens from the infected animal or human to new hosts or locations.

In the laboratory, conventional PCR tests have a turnaround time of minimum 3 hours. Faster accurate tests results are highly desirable in particular settings, such as for instance at the level of pre-departure testing at airports or pre-event testing.

Thus, it is a problem that detection or quantitative analysis of certain infections, such as Covid-19, takes long time, which increases waiting times for test persons and may increase risks of contamination of further individuals.

It is a further problem that detection or quantitative analysis of certain viral infections, such as Coronavirus, often is associated with large laboratory equipment.

### Summary of the invention

It is an object to, at least partly, mitigate, alleviate, or eliminate one or more of the above-identified deficiencies in the art and disadvantages singly or in any combination and solve at least the above-mentioned problem.

It is a particular object to provide a system for nucleic acid analysis, which system at least partly, mitigate, alleviate, or eliminate one or more of the above-identified deficiencies in the art.

It is a further particular object to provide a system for PCR analysis, which system at least partly, mitigate, alleviate, or eliminate one or more of the above-identified deficiencies in the art.

In the following, the nucleic acid analysis will be described and exemplified with PCR analysis. Other types of nucleic acid analysis may be used alternatively.

According to a first aspect there is provided a system for PCR analysis. The system comprises:
a test card receiver configured to receive a test card, wherein the test card comprises a sample portion having a first side and a second side opposite the first side, wherein the sample portion of the test card is configured to hold a sample for PCR,
a temperature adjuster configured to adjust a temperature of the sample portion of the test card over a plurality of temperature cycles, wherein each temperature cycle is between a first temperature and a second temperature different than the first temperature, wherein the temperature adjuster comprises a surface configured to be in contact with the first side of the sample portion of the test card,
wherein the test card receiver comprises a spring member configured to push the sample portion of the test card towards the surface of the temperature adjuster, and
a monitoring arrangement configured to monitor the sample portion of the test card over the plurality of temperature cycles, the monitoring arrangement comprising:
   at least one light sensor,
   an illumination system configured to illuminate the second side of the sample portion with light having wavelengths within at least three excitation wavelengths ranges,
   an emission filter configured to transmit light having wavelengths within at least three emission wavelength ranges, and
   collection optics configured to collect light emanating from the second side of the sample portion of the test card and transmitted through the emission filter, and wherein the collection optics is further configured to direct the collected light towards the light sensor such that at least a portion of the collected light impinges on the light sensor,
   wherein the light sensor is configured to generate at least one signal, wherein each signal pertains to light impinging on the light sensor after a unique number of temperature cycles of the plurality of temperature cycles.

As used herein, the term "sample" or "sample for PCR", is to be construed broadly as any liquid potentially comprising an analyte of interest, which liquid can be provided into the test card for being processed on said test card. As used herein, the analyte present in the liquid will usually be a target nucleic acid to be detected with the help of the test card being engageable in the present system for nucleic acid analysis. The sample can be liquid biological sample, e.g. blood, obtained from an individual, but it can also be a biological sample, e.g. a nasopharyngeal or throat swab sample, that was preprocessed on a bench in accordance with any sample processing protocol, e.g. a nucleic acid extraction protocol. The liquid may further contain all the reagents required to amplify and detect specific nucleic acid analytes that may be present in such biological sample. The nucleic acid in the liquid may then be directly analyzed for its presence using the disclosed herein system for nucleic acid analysis.

As used herein the term "analyte" is to be construed as a term synonymous to a term "biomarker" or "target", which terms relate to a detectable nucleic acid fragment of interest, usually a nucleic acid fragment of a pathogen, e.g. viral DNA or RNA.

As used herein, the term "PCR" is to be construed broadly as any technique dealing with polymerase chain reaction and will be understood as also comprising RT-qPCR.

As used herein, the term "plurality" in "plurality ..." is to be understood as referring to more than one, e.g. a plurality of temperature cycles. In the context of "plurality of temperature cycles", the term "plurality" will usually refer to more than 1, such as, 2, 3, 4, 5, 6, 7, 8, 9, 10 or in the range of multiples of 2, 5 or 10, or 5 to 60 cycles, or 45 to 55 cycles.

The test card receiver allows the system to be used with a test card. The test card comprises a sample portion, which may be a sample handling portion, such as a microfluidic chip, allowing sample to be handled and subjected to PCR. The test card comprising the sample portion allows desirable sample handling due to the sample portion, while at the same time allowing a suitable shape and structure of the test card, thus allowing desirable handling, such as handling prior to and during insertion of the test card into the system.

The temperature adjuster allows adjusting the temperature of the sample portion of the test card, which is important in PCR. Adjusting the temperature of the sample portion of the test card over a plurality of temperature cycles, wherein each temperature cycle is between a first temperature and a second temperature different than the first temperature, thus allowing denaturation and annealing in PCR.

The temperature adjuster comprising a surface configured to be in contact with the first side of the sample portion of the test card enables efficient thermal contact and heat transfer between the temperature adjuster and the sample portion, thus providing benefits for PCR within the sample portion.

The test card receiver comprising a spring member configured to push the sample portion of the test card towards the surface of the temperature adjuster, allows for providing efficient thermal contact and rapid heating/cooling of the sample portion. Further, the spring member allows for efficient insertion and removal of the test card in the system. The spring member further allows for provision of suitable and desirable push force of the sample portion against the surface of the temperature adjuster, for example allowing for not just providing thermal contact, but also suitable forces such that damage is not inflicted on the sample portion. This may be beneficial, for example, if the sample portion is a silica chip, or of other brittle material. The spring member further allows optimization between high thermal contact while avoiding damage to the test card or the sample portion of the test card.

The monitoring arrangement configured to monitor the sample portion of the test card over the plurality of temperature cycles allows efficient monitoring of the PCR, taking place within or on the sample portion. For example, the PCR may be monitored over time and real-time PCR monitoring may be realised. In an alternative setting, the PCR may be monitored at the end-point stage or a stage in-between PCR start and end-point.

Having at least one light sensor allows sensing or detecting light from the PCR, such as fluorescence generated during the PCR.

The illumination system being configured to illuminate the second side of the sample portion, while at the same time the temperature adjuster comprises a surface configured to be in contact with the first side of the sample portion, provides for a desirable design of the system. For example, the temperature adjuster may be positioned such that interference of the illumination is reduced or avoided.

The light having wavelengths within at least three excitation wavelengths ranges, allows for efficient detection or monitoring of the PCR. For example, at least three different markers or fluorophores may be used. Further, simultaneous analysis or detection of at least three different DNA sequences may be realised.

The emission filter being configured to transmit light having wavelengths within at least three emission wavelength ranges, allows for at least three different emitted signals to be detected. The emission filter being configured to transmit light having wavelengths within at least three emission wavelength ranges may be realised, for example, by an emission filter being composed of at least three sub-emission filters.

The collection optics configured to collect light emanating from the second side of the sample portion of the test card and transmitted through the emission filter, and wherein the collection optics is further configured to direct the collected light towards the light sensor such that at least a portion of the collected light impinges on the light sensor, allows detection or sensing of light, such as fluorescence, from the sample portion, and thus, for example, monitoring of PCR within the sample portion.

The light sensor being configured to generate at least one signal, wherein each signal pertains to light impinging on the light sensor after a unique number of temperature cycles of the plurality of temperature cycles allows efficient monitoring of the PCR.

The system for PCR analysis may be a system for nucleic acid analysis.

The system for PCR analysis may be a system for nucleic acid analysis using PCR or RT-PCR.

The nucleic acid may be DNA or RNA, or sequences thereof.

The at least one light sensor may be at least one image sensor, and the collection optics may be imaging optics configured to image the sample portion on the image sensor.

Thereby, imaging of the sample portion and the PCR is enabled.

The monitoring arrangement may comprise:
the at least one light sensor
a first light source configured to illuminate the second side of the sample portion through a first excitation light filter, thereby providing light having wavelengths within a first excitation wavelength range at the second side of the sample portion of the testcard,
a first emission filter configured to transmit light having wavelengths within a first emission wavelength range,
a first collection optics configured to collect light emanating from the second side of the sample portion of the test card and transmitted through the first emission filter, and wherein the collection optics is further configured to direct the collected light towards the light sensor such that at least a portion of the collected light impinges on the light sensor,
a second light source configured to illuminate the second side of the sample portion through a second excitation light filter, thereby providing light having wavelengths within a second excitation wavelength range at the second side of the sample portion of the testcard,
a second emission filter configured to transmit light having wavelengths within a second emission wavelength range,
a second collection optics configured to collect light emanating from the second side of the sample portion of the test card and transmitted through the second emission filter, and wherein the collection optics is further configured to direct the collected light towards the light sensor such that at least a portion of the collected light impinges on the light sensor,
a third light source configured to illuminate the second side of the sample portion through a third excitation light filter, thereby providing light having wavelengths within a third excitation wavelength range at the second side of the sample portion of the testcard,
a third emission filter configured to transmit light having wavelengths within a third emission wavelength range, and
a third collection optics configured to collect light emanating from the second side of the sample portion of the test card and transmitted through the third emission filter, and wherein the collection optics is further configured to direct the collected light towards the light sensor such that at least a portion of the collected light impinges on the light sensor.

With such a system three, or more, i.e. a first, a second and a third, excitation/emission reactions, or more, may be monitored. This allows, for example, at least three different analyte sequences or portions of analyte sequences, or e.g. DNAs, to be monitored, for example one or more of a plurality of sequences relating to COVID-virus, relating to a "background reaction", and relating to one or more of other type of viruses or analytes.

The at least one light sensor may be at least one imaging sensor, and each of the collection optics may be a respective imaging optics configured to collect light transmitted through the emission filter and to image the second side of the sample portion of the test card on the least one image sensor.

Thereby, monitoring, such as end-point or real-time monitoring of PCR within the sample portion may be realised. Further, other types of reactions or occurrences within the sample portion may be followed and monitored.

The at least one image sensor may be configured to capture at least one image of the second side of the sample portion, wherein each image is captured after a unique number of temperature cycles of the plurality of temperature cycles.

Thereby, for example, an entire or part of a PCR may be monitored or imaged at desirable frequencies of images.

The at least one light sensor may be at least one photodiode.

By using photodiode(s), assembly tolerance can be relaxed. For example, a need for keeping a focal plane over the sample can be avoided. Further, a system comprising photodiode(s) can have a small size. Yet further, data to be analysed can be reduced in size, for example, as compared to data relating to images.

Each of the first light source, the second light source, and the third light source may be a LED.

Thereby, narrow bandwidth of excitation light may be used with the system. Further, an LEDs are readily available, and are of low maintenance.

The spring member may be configured to push the sample portion of the test card towards the surface of the temperature adjuster at a force of 5-15 N, such as 8-12 N.

Thereby, efficient thermal contact between the sample portion and the temperature adjuster is realised, while at the same time damages or risks of damage to the sample portion or testcard is minimised or reduced, in particular when a silicon chip is used as sample portion.

The spring member may comprise a leaf spring. The leaf spring may be configured to allow transmitting light to and from the second side of the sample portion by having a slit or aperture.

Thereby, even when the spring member is positioned on or adjacent to the second side of the sample portion, the spring member can be arranged such that it does not obscure the sample portion from the sensor system. The temperature adjuster may comprise a thermoelectric element. The thermoelectric element may be a Peltier element.

With a thermoelectric element, such as a Peltier element, heating and/or cooling may be achieved in efficient manner. Thereby, a plurality of consecutive temperature cycles may be achieved.

One of the first temperature and the second temperature may be within the interval 60-70°C and the other of the first temperature and the second temperature may be within the interval 90-105°C.

Thereby, suitable temperature cycles, or denaturation and annealing cycles, for PCR may be achieved.

The temperature adjuster may be configured to provide 1 to 100 number of temperature cycles, such as 5-60, or 45-50 number of temperature cycles.

Thereby, suitable number of denaturation and annealing cycles for PCR may be achieved.

Each temperature cycle may have a duration of 20 seconds or below; or 15 seconds or below.

With such temperature cycle durations, it is possible to achieve PCR with the system according to the first aspect or embodiments.

The light sensor may be configured to generate at least one signal, per temperature cycle over at least 5 number of temperature cycles, or 10 number of temperature cycles, or 45-55 number of temperature cycles.

Thereby, the PCR may be monitored over time and real-time PCR is achievable.

The first excitation wavelength range, the second excitation wavelength range, and the third excitation wavelength range may be different wavelength ranges.

Thereby, for example, a first, a second, and a third sequence or unique DNA may be monitored. Further, for example, three different viruses may be analysed, or three different type of fluorophores may be monitored.

The first emission wavelength range, the second emission wavelength range, and the third emission wavelength range are different wavelength ranges.

Thereby, for example, a first, a second, and a third sequence or unique DNA may be monitored. Further, for example, three different viruses may be analysed, or three different type of fluorophores may be monitored.

The first side and the second side of the sample portion may be parallel to a main side of the test card.

Thus, for example, a flat test card may be used.

According to a second aspect there is provided a method for performing PCR analysis using the system according to the first aspect, the method comprising:
receiving a test card at a test card receiver, wherein the test card comprises a sample portion having a first side and a second side opposite the first side, wherein the sample portion of the test card holds a sample for PCR,
adjusting a temperature of the sample portion of the test card (6) over a plurality of temperature cycles, wherein each temperature cycle is between a first temperature and a second temperature different than the first temperature, using a temperature adjuster comprises a surface being in contact with the first side of the sample portion of the test card,
wherein the test card receiver comprises a spring member pushing the sample portion of the test card towards the surface of the temperature adjuster, and
monitoring the sample portion of the test card over the plurality of temperature cycles, using a monitoring arrangement comprising:
   at least one light sensor,
   an illumination system illuminating the second side of the sample portion with light having wavelengths within at least three excitation wavelengths ranges,
   an emission filter transmitting light having wavelengths within at least three emission wavelength ranges, and
   collection optics collecting light emanating from the second side of the sample portion of the test card and transmitted through the emission filter, and wherein the collection optics further direct the collected light towards the light sensor wherein at least a portion of the collected light impinges on the light sensor,
   wherein the light sensor generates at least one signal, wherein each signal pertains to light impinging on the light sensor after a unique number of temperature cycles of the plurality of temperature cycles.

According to a third aspect, there is provided a use of the system according to any one of claims the first aspect, for PCR analysis or nucleic acid analysis.

The above-mentioned features relating to one of the aspects apply, when relevant, also to another of the aspects. In order to avoid undue repetition, reference is made to the above.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description. Hence, it is to be understood that this inventive concept is not limited to the particular steps of the methods described or component parts of the systems described as such method and system may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

### Brief description of the drawings

The above and other aspects of the present inventive concept will now be described in more detail, with reference to appended drawings showing variants of the inventive concept. The figures should not be considered limiting the inventive concept to the specific variant; instead, they are used for explaining and understanding the inventive concept. As illustrated in the figures, the sizes of layers and regions are exaggerated for illustrative purposes and, thus, are provided to illustrate the general structures of variants of the present inventive concept. Like reference numerals refer to like elements throughout.
Figure 1 illustrates a system according to an embodiment.
Figure 2 illustrates a system according to an embodiment.
Figure 3 illustrates a system according to an embodiment.
Figure 4 illustrates an embodiment comprising a leaf spring.
Figure 5 illustrates a system comprising a housing according to an embodiment.
Figure 6 illustrates a temperature profile during a temperature cycle according to an embodiment.
Figure 7 illustrates a method according to an embodiment.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

With systems described herein, nucleic acid analysis may be performed and monitored. Further, PCR may be performed and monitored. It will be understood from below how this may be achieved. The temperature adjuster allows the necessary heating and cooling used for denaturation and annealing of eg. DNA. Thereby, DNA and RNA may be analysed, the RNA via preceding transcription to cDNA. Light sources, light filters, collection optics and light sensor allows monitoring of the PCR through, for example, use of fluorophores on the DNA or RNA, which fluorophores may be excited and emit light, which emitted light may be detected by the light sensor. The PCR may, thus, be monitored using the system. The actual PCR takes place on the sample portion of the test card. It is, thus, assumed that the sample portion comprises, or can receive, a sample comprising eg. RNA or DNA, reagents for PCR, and any necessary liquids or buffers, etc.

PCR chemistries and detection formats compatible to the systems of the present invention herein have been described. These include non-specific detection formats such as intercalating dyes such as double-stranded DNA binding dyes, e.g. CYBR Green. The use of hairpin primers which emit a fluorescent signal when unfolded during its incorporation into an amplification product have been described, e.g. Amplifluor technology. Sequence-specific detection formats yielding a measurable fluorescence emission include probes being homologous to an internal region amplified by two PCR primers. They can be classified into two major groups: hydrolysis probes, e.g. TaqMan probes, TaqMan MGB probes, and hybridizations probes, e.g. FRET hybridization probes, Molecular Beacons, Scorpion primers, etc. Fluorescence signal intensity can be related to the amount of PCR product (i) by a product-dependent decrease of the quench of a reporter fluorophore or (ii) by an increase of the fluorescence resonance energy transfer (FRET) from a donor to an acceptor fluorophore. Commonly used fluorescent reporter dyes are FAM, TET (tetrachloro-6-car-boxyfluorescein), JOE (2,7-dimethoxy-4,5-dichloro-6-carboxy-fluorescein) or HEX (hexacholoro-6-carboxyfluorescein), and frequently used quenchers are TAMRA, DABCYL and Black Hole Quencher (BHQ). Other technologies use a nucleic acid analogue instead of natural nucleic acid sequence recognition element, e.g peptide nucleic acids (PNAs).

Although PCR and RT-PCR are most-known exemplary nucleic acid amplification methods in the field, alternative methods such as LAMP, NASBA, etc. may also be used in different embodiments of the systems described herein as compatible therewith.

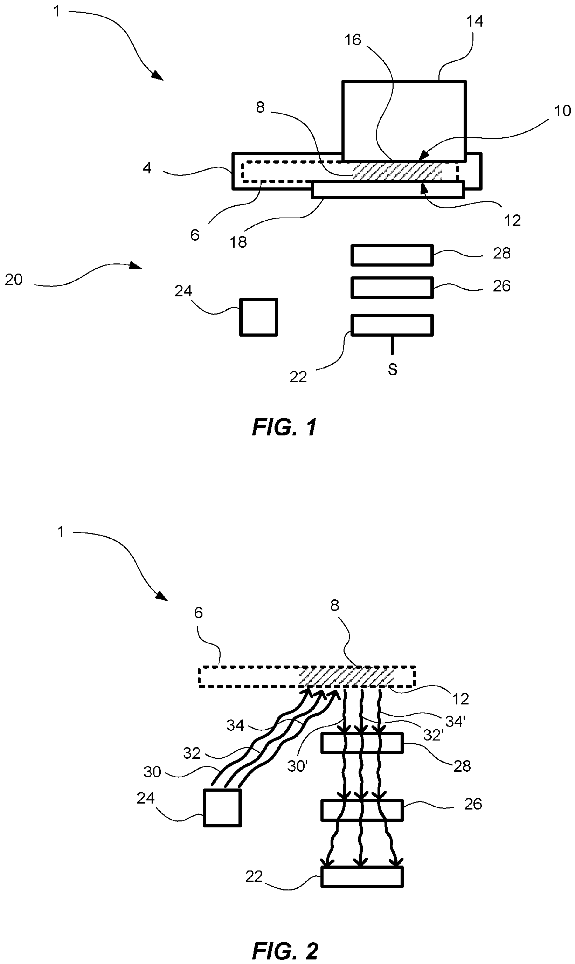

With reference to figure 1, a system 1 for PCR analysis will now be discussed. It shall be understood that figure 1 is a schematic illustration illustrating components of the system 1. The system 1 comprises:
a test card receiver 4 configured to receive a test card 6. The test card 6 is illustrated in dotted lines to illustrate that it is not part of the system 1 in the illustrated embodiment. The test card 6 comprises a sample portion 8 having a first side 10 and a second side 12 opposite the first side, wherein the sample portion 8 of the test card is configured to hold a sample for PCR. The sample for PCR may be a sample comprising DNA or RNA, or sequence(s) thereof. The system 1 further comprises a temperature adjuster 14 configured to adjust a temperature of the sample portion 8 of the test card 6 over a plurality of temperature cycles, wherein each temperature cycle is between a first temperature and a second temperature different than the first temperature, wherein the temperature adjuster 14 comprises a surface 16 configured to be in contact with the first side 10 of the sample portion 8 of the test card 6.

The test card receiver 4 comprises a spring member 18 configured to push the sample portion 8 of the test card 6 towards the surface 16 of the temperature adjuster 14. The system 1 further comprises a monitoring arrangement 20 configured to monitor the sample portion 8 of the test card 6 over the plurality of temperature cycles. The monitoring arrangement 20 comprises: at least one light sensor 22; an illumination system 24 configured to illuminate the second side 12 of the sample portion 8 with light having wavelengths within at least three excitation wavelengths ranges; an emission filter 26 configured to transmit light having wavelengths within at least three emission wavelength ranges; and collection optics 28 configured to collect light emanating from the second side 12 of the sample portion 8 of the test card 6 and transmitted through the emission filter 26, and wherein the collection optics 28 is further configured to direct the collected light towards the light sensor 22 such that at least a portion of the collected light impinges on the light sensor 22, wherein the light sensor 22 is configured to generate at least one signal S, wherein each signal S pertains to light impinging on the light sensor 22 after a unique number of temperature cycles of the plurality of temperature cycles.

In addition to the sample portion 8 of the test card being configured to hold a sample for PCR, such as at least one DNA or RNA molecule, the sample portion may be configured to hold reagents for PCR, and necessary liquids and/or buffers.

It will be appreciated that the positioning of eg. the emission filter 26 and the collection optics 28 in relation to each other, and to the test card 6 and the light sensor 22 may be different from the illustrated. The positioning of the emission filter 26 and the collection optics 28 may be interchanged. The positioning or order from the test card 6 to the light sensor 22, may be test card 6 - collection optics 28 - emission filter 26 - light sensor 22, as illustrated, or test card 6 - emission filter 26 - collection optics 28 - light sensor 22.

The test card receiver 4 may be any suitable means for receiving and/or holding the test card 6. The test card 6 may be received in a position fixed in relation to the monitoring arrangement 20, or in a fixed position in relation to the monitoring arrangement 20 and the temperature adjuster 14.

The test card receiver 4 may be a suitable space within the system sufficiently large to hold the test card 6. For example, the system may comprise rails or guides, or other suitable means, used to allow insertion and holding the test card 6. Thereby, the sample portion of the test card may be guided and aligned to the surface of the temperature adjuster. Thereby, suitable or optimal contact between the two may be realised. In addition, the sample portion may be suitably positioned in relation to a focal plane of eg. a camera, such as if imaging with eg. a camera is used.

The test card receiver 4 allows the system 1 to be used with a test card 6. The test card 6 comprises a sample portion, which may be a sample handling portion, such as a microfluidic chip, allowing sample to be handled and subjected to PCR.

According to one example the test card 6 may be, for example 10-100 mm long, 5-50 mm wide, and 0.1-10 mm thick. Such a test card 6 may efficiently be handled during sample loading and insertion into the system. The sample portion 8, for example in form of a chip, may have a major surface parallel to a major surface of the test card smaller than 15² mm², such as, for example, 15x15 mm² or smaller; 12x12 mm², or smaller; such as 5x10 mm², or smaller.

In an alternative embodiment, the monitoring arrangement may comprise: at least one light sensor; an illumination system 24 configured to illuminate the second side of the sample portion with light having wavelengths within at least two, or at least one, excitation wavelength ranges; an emission filter 26 configured to transmit light having wavelengths within at least two, or at least one, emission wavelength ranges. Such an illumination system may comprise, for example, two, or one, light sources.

To improve understanding of the system and the monitoring arrangement 20, figure 2 will now be discussed. Figure 2 illustrates part of the system 1, with parts left out in an attempt to improve clarity and understanding of the following discussion. The system 1 may be identical to the system 1 as illustrated and discussed with reference to figure 1. In figure 2 the illumination system 24, which, for example, may comprise at least three light sources, such as light emitting diodes (LEDs), is configured to provide three different excitation wavelength ranges, schematically simplified and illustrated using three arrows 30, 32, 34, and to illuminate the second side 12 of the sample portion 8 of the test card 6. For example, light sources of the illumination system 24 may be directed towards the second side 12 of the sample portion 8. The light sources of the illumination system may be configured to illuminate the sample portion sequentially or simultaneously. The collection optics 28 collect light emanating from the second side 12 of the sample portion 8 of the test card 6 and the light (schematically simplified and illustrated using three arrows 30', 32', 34') is transmitted through the emission filter 26. Thereby, the emission filter 26 is allowed to filter out light which is not of interest, such as light which is not associated with a fluorescence reaction of interest relating to the corresponding excitation wavelength. The collection optics 28 direct the collected light towards the image light sensor 22 such that at least a portion of the collected light impinges on the light sensor 22. For example, the collection optics 28 may comprise at least three lenses, one for each emission wavelength range, which may direct each emission wavelength range towards a respective portion of the light sensor 22. It shall be realised that the system 1, thus is suitable for monitoring of PCR taking place within the sample portion 8 of the test card 6. For example, each excitation wavelength range may act to excite a fluorophore of a unique DNA/RNA sequence, for example associated with a unique virus or unique part of a virus. Light emitted by a respective fluorophore may then be filtered and directed towards and sensed by the light sensor 22, which allows for monitoring of the PCR of three unique sequences, or, for example, three different viruses.

The emission filter 26 being configured to transmit light having wavelengths within at least three emission wavelength ranges may be realised, for example, by an emission filter being composed of, or comprising, at least three sub-emission filters.

The collection optics 28 being configured to collect light emanating from the second side 12 of the sample portion 8 of the test card 6 and transmitted through the emission filter 26, allows the collection optics to be associated with each emission wavelength range individually. The collection optics 28 being further configured to direct the collected light towards the light sensor 22, such that at least a portion of the collected light impinges on the light sensor 22, allows each emission wavelength range to be associated with an individual portion of the light sensor 22.

The signal S generated by the light sensor 22 may pertain to an intensity of light impinging on the light sensor. Suitable software and/or controller may be integrated with the system 1 or provided outside of the system and used for the purpose of handling the sensor, analysing the at least one signal, and/or handling imaging.

In a case where the light sensor is an image sensor, and the collection optics is imaging optics, the signal may pertain to an image of at least a portion of the second side 12 of the sample portion 8. The image may be a colour image or a grayscale image.

The sample for PCR may be a sample comprising or consisting of at least one nucleic acid molecule, such as DNA or RNA.

The sample portion being configured to hold a sample for PCR may be achieved by the sample portion comprising a channel, such as a microfluidic or capillary channel, or a sample well or compartment, capable of holding a sample, wherein the sample may comprise DNA or RNA, or at least a fragment thereof, and, optionally, any eg. liquids, for example buffers, and reagents needed for the PCR. For example, the sample portion 8 may be a chip, such as a silica chip, which, thus, may comprise a channel, or compartment, or a well, or combinations of two or more of those.

RNA of the sample may be converted to cDNA for the analysis.

The at least one light sensor may be at least one image sensor, and the collection optics may be imaging optics configured to image the sample portion on the image sensor.

Thereby, imaging of the sample portion and the PCR is enabled.

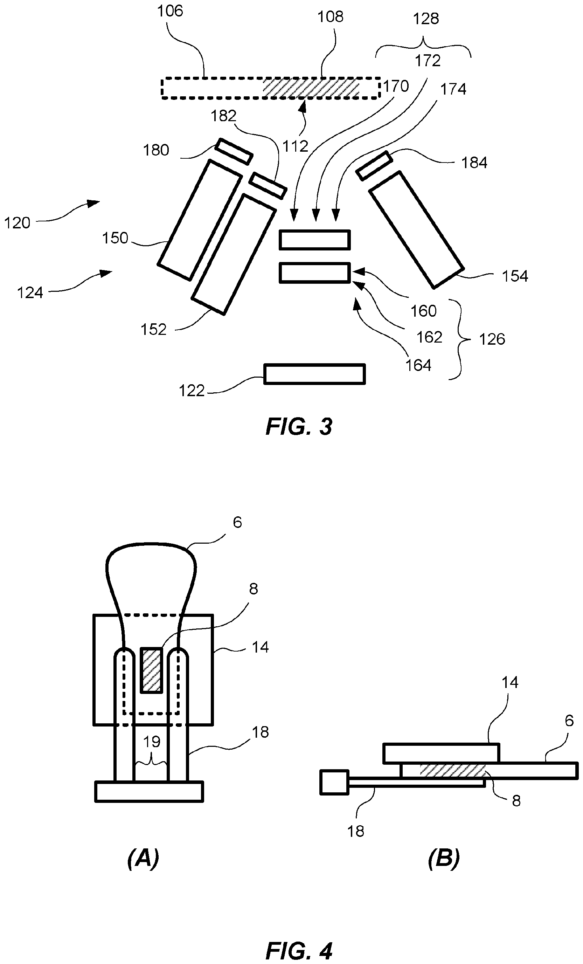

With reference to figure 3, a system 100 according to an example or embodiment will now be discussed in further detail. The system 100 comprises components discussed with reference to figure 1, but is further detailed as described below. Temperature adjuster 14, test card receiver 4, and spring member 18 is left out from the illustration in figure 3 in an attempt to improve clarity, it shall be realised that they, for example, may be positioned as illustrated in figure 1. The monitoring arrangement 120 is illustrated and comprises the at least one light sensor 122. The monitoring arrangement 120 further comprises an illumination system 124 comprising a first light source 150, a second light source 152, and a third light source 154; an emission filter 126 comprising a first emission filter 160, a second emission filter 162, and a third emission filter 164. Collection optics 128, of the monitoring arrangement 120, comprises a first collection optics 170, a second collection optics 172, and a third collection optics 174.

The first light source 150 is configured to illuminate the second side 112 of the sample portion 108 through a first excitation light filter 180, thereby providing light having wavelengths within a first excitation wavelength range at the second side 112 of the sample portion 108 of the testcard 106. The first emission filter 160 is configured to transmit light having wavelengths within a first emission wavelength range, and the first collection optics 170 is configured to collect light emanating from the second side 112 of the sample portion 108 of the test card 106 and transmitted through the first emission filter 160, and wherein the first collection optics 170 further is configured to direct the collected light towards the light sensor 122 such that at least a portion of the collected light impinges on the light sensor 122.

The second light source 152 is configured to illuminate the second side 112 of the sample portion 108 through a second excitation light filter 182, thereby providing light having wavelengths within a second excitation wavelength range at the second side 112 of the sample portion 108 of the testcard 106. The second emission filter 162 configured to transmit light having wavelengths within a second emission wavelength range, and the second collection optics 172 is configured to collect light emanating from the second side 112 of the sample portion 108 of the test card 106 and transmitted through the second emission filter 162, and wherein the collection optics is further configured to direct the collected light towards the light sensor 122 such that at least a portion of the collected light impinges on the light sensor 122.

The third light source 154 is configured to illuminate the second side 112 of the sample portion 108 through a third excitation light filter 184, thereby providing light having wavelengths within a third excitation wavelength range at the second side 112 of the sample portion 108 of the testcard 106. The third emission filter 164 is configured to transmit light having wavelengths within a third emission wavelength range, and the third collection optics 174 is configured to collect light emanating from the second side 112 of the sample portion 108 of the test card 106 and transmitted through the third emission filter 164, and wherein the third collection optics 184 is further configured to direct the collected light towards the light sensor 122 such that at least a portion of the collected light impinges on the light sensor 122.

With such a system, three, or more, i.e. a first, a second and a third, excitation/emission reactions, or more, may be monitored. This allows, for example, three different sequences or portions of sequences, or DNAs, to be monitored, for example one or more of a plurality of sequences relating to COVID-virus, relating to a control sample, and relating to one or more of other type of viruses.

The emission filter, such as the first, the second, and the third emission filters, may, in addition to being configured to transmit light having wavelengths within the first, the second, and the third emission wavelength range, respectively, further be configured to block a portion of light having wavelengths outside the respective wavelength range.

The light sensor 122 may comprise a plurality of light sensors. For example, there may be a first, a second, and a third light sensor, one for each of the first, second and third collection optics. Further, the light sensor 122 may be one light sensor divided in portions of which one or more portions corresponds to a respective emission wavelength range.

The system 100 may be used for imaging of the sample portion 8 of the test card 6, or a portion of the sample portion 8, and any PCR within the sample portion 8 of the test card 6. In such a system 100, similar to the system 100 discussed with reference to figure 3, the at least one light sensor 122 may be at least one imaging sensor 123, and each of the collection optics 128 may be a respective imaging optics configured to collect light transmitted through the emission filter 126 and to image the second side 112 of the sample portion 108 of the test card 106 on the least one image sensor 122.

Thereby, monitoring, such as end-point or real-time monitoring of PCR within the sample portion may be realised. Further, other types of reactions or occurrences within the sample portion may efficiently be followed and monitored.

The at least one imaging sensor 122, may be, for example, a CMOS or a CCD sensor.

The at least one image sensor may be configured to capture at least one image of the second side of the sample portion, wherein each image is captured after a unique number of temperature cycles of the plurality of temperature cycles.

This shall be understood as each image, of the at least one image, is being captured after a number of temperature cycles, the number being unique for each of the at least one image. Put differently, if eg. three images are captured, i.e. a first, a second, and a third image, each of the three images are captured after a respective number of temperature cycles. For example, the first image may be captured after one temperature cycle, the second image may be captured after 10 image cycles, and the third image may be captured after 20 temperature cycles. According to another example, one image may be captured after 1 temperature cycle, another image may be captured after 2 temperature cycles, and a third image may be captured after 3 temperature cycles. To provide further examples, an image may be captured after each of the temperature cycles, after every tenth temperature cycle, etc.

For a temperature cycle, the at least one image may be captured sequentially or simultaneously. For example, if more than one image sensor is used.

In case the at least one light sensor is at least one image sensor, each signal of the at least one signal may be an image of the second side of the sample portion.

Thereby, an entire or part of a PCR may be monitored or imaged at desirable frequencies of images. It may be that it is decided to monitor at the beginning and at the end of a PCR with a predetermined number of cycles. It may be that the beginning of the PCR is closely monitored for determining when the number of copies are increasing rapidly, or it may be that the end of the PCR is monitored, for determining when sufficient number of copies have been obtained to allow for desired analysis, and/or termination of the PCR.

The at least one light sensor may be at least one photodiode. The use of collection optics may direct each emission wavelength range towards a respective portion of the light sensor or image sensor, thus still allowing separate analysis of each emission wavelength range.

Each of the first light source, the second light source, and the third light source may be a LED.

Thereby, narrow bandwidth of excitation light may be used with the system. Further, an LEDs are readily available, and are of low maintenance.

Alternatively, each of the first light source, the second light source, and the third light source may be a LASER.

Further, the light sources may comprise both LED and LASER.

The spring member may be configured to push the sample portion of the test card towards the surface of the temperature adjuster at a force of 5-15 N, such as 8-12 N.

Thereby, efficient thermal transfer between the sample portion and the temperature adjuster is realised, while at the same time minimizes damages or risks of damage to the sample portion or testcard, in particular when a silicon chip is used as sample portion.

According to an embodiment, a silica or a glass chip comprises the sample portion 8. The part of the test card not constituting the chip or sample portion 8 may be manufactured from plastic.

The spring member may contact the sample portion 8 or the test card 6 on a side adjacent to or coinciding with the second side of the sample portion.

The tension or force between the sample portion, or chip, of the testcard and the temperature adjuster has influence on the heat transition. The force onto the testcard may, as described, be regulated using a leafspring.

The leafspring may be manufactured as long as is needed to be tolerant to force differences by make- or assembly processes. For example, the leafspring may result in a nomical value of 10N with 0.6N/mm force built-up in the leafspring. Tips of the leafspring may be bend to prevent deflection of testcard. The bend tips may further enable efficient sliding of the testcard over the leafspring, thus providing gaining a pretention when the testcard is inserted.

The leafspring may be plasma polished to minimise friction and debris from the testcard, thereby reducing risk of pollution of the system, such as of optical paths.

The force may, for example, thus, be presented to the sample portion, or the test card, in two steps, which may be according to the below examples 1) and 2):
1) The temperature adjuster is in an up position (such as by being fastened to a lid of a housing of the system, the lid being in an open position), and the test card is slid into the test card receiver resulting in 0.9 mm deflection on springs equaling 5.6 N in springs
2) The temperature adjuster is in a down position (such as by being fastened to a lid of a housing of the system, the lid being in a closed position). The temperature adjuster then pushes the sample portion or the test card towards the leafspring resulting in, for example, 1.6 mm deflection equaling 10 N in leafspring.

A contact area between the temperature adjuster and a sample portion, in this example in form of a silicon chip having a size of 5.75 mm x5mm, or 28.75 mm², which results in 2.88 N/mm² contact force.

Force on the testcard may, thus, be controlled not to be too high, since movement of the temperature adjuster in contact with the sample portion, or chip, may be restricted and the leafspring being deflect under pressure.

The spring member 18 may comprise a leaf spring configured to allow transmitting light to and from the second side of the sample portion by having a slit or aperture or gap. Figure 4 illustrates an example of such spring member 18 being a leaf spring, in a part of the system 1. Figure 4(A) illustrates a bottom view and figure 4(B) illustrates a side view. The test card 6, comprising the sample portion 8 is clamped between the spring member 18 and the temperature adjuster 14. A slit or aperture of the spring member 18 is illustrated and indicated with parenthesis 19. It shall be apparent that the slit or aperture 19 allows light to enter and exit, or illuminate to and from, the sample portion 8 of the test card 6 from the side of the test card where the spring member 18 is positioned.

Thereby, as exemplified, even when the spring member is positioned on or adjacent to the second side of the sample portion, the spring member can be arranged such that it does not obscure the sample portion from the sensor system.

The temperature adjuster may comprise a thermoelectric element. The thermoelectric element may be a Peltier element.

The temperature adjuster may further comprise a heatsink, which may be combined with a fan. Thereby cooling time of the sample portion may be reduced, and thereby the overall time for the temperature cycles and the PCR may be reduced. The heatsink may be a heatsink known in the art, such as using eg. flanges or other type of protrusions and, optionally, a fan.

With a thermoelectric element, such as a Peltier element, heating and/or cooling may be achieved and controlled in an efficient manner and a plurality of consecutive temperature cycles may be achieved.

The thermoelectric element, or peltier element, may have a hot side and a cold side of aluminum, which allows high power throughput and provides good shapability for good thermal contact. In combination with a silicon or glass chip comprising the sample portion, which silicon or glass chip allows high temperatures, the heating cycles can be rapid.

One of the first temperature and the second temperature may be within the interval 60-70°C and the other of the first temperature and the second temperature may be within the interval 90-105°C.

Thereby, suitable temperature cycles, or denaturation and annealing cycles, for PCR may be achieved. In particular, those temperature ranges is suitable for PCR for COVID analysis.

The heating cycles does not exclude that other heating and/or cooling steps are used in addition to the heating cycles. For example, such as for COVID PCR analysis, a first reverse transcriptase (RT) step at a temperature suitable for the reverse transcriptase activity of the enzyme may be conducted on the nucleic acid present in the sample, for example at 60-62°C, such as 61°C, followed by the heating cycles. This principle is known as RT-PCR.

For example, prior to temperature cycles, any RNA present in sample may be converted to DNA, which may take place, for example, during a time period of about a minute, i.e. within the interval 30 to 90 seconds.

Of the temperature cycle, the first temperature may be the higher temperature and the second temperature may be the lower temperature. The first and higher temperature may correspond to or result in denaturation of the nucleic acid in the PCR, and the second and lower temperature may correspond to or result in annealing of the nucleic acid in the PCR.

The temperature cycles are described using two temperatures, the first and the second temperatures. It will be understood that a cycle implies not just going from a first temperature to the second temperature, but in addition going again to the first temperature. However, as long as the temperature adjuster of the system adjust the temperature over a plurality of temperature cycles, it is not excluded that a not complete cycle is preceding or following the cycles. For example, PCR may follow the following examples of heat adjustments and be within embodiments herein: T1-T2-T1-T2-T1 (two cycles); or T2-T1-T2-T1-T2-T1 (two cycles preceded by T2); or T1-T2-T1-T2-T1-T2 (two cycles followed by adjusting to the second temperature).

It shall be realised that the cycle profile does not have to be symmetrical, although it may be symmetrical, thus, the first and the second temperatures do not have to be held for equally long times, although it is not excluded that they do. For example, the higher temperature may be held for a shorter time than the lower temperature. This may be beneficial and speed up the cycling time, as the denaturing may occur quicker than the annealing. The higher temperature may, alternatively, be held for a longer, or equal, time than the lower temperature.

The temperature adjuster being configured to adjust the temperature of the sample portion may be by heating the surface of the temperature adjuster to 1-5°C above the first and second temperatures, respectively. Thereby it has been found that the desired temperature within the sample portion of the test card may be achieved in a rapid manner. Settings a controlling of the temperature adjuster can be decided, for example, using experiments and measurements incorporating the sample portion and the temperature adjuster.

The temperature adjuster may be configured to provide 1 to 100 number of temperature cycles, such as 5-60, or 45-50 number of temperature cycles. Further, the temperature adjuster may be configured to provide 5-60, 10-60, 15-60, 20-60, 25-60, 30-60, 40-60, 40-55, 41-54, 42-53, 43-52, 44-51, 45-50 number of temperature cycles.

Thereby, suitable number of denaturation and annealing cycles for PCR may be achieved.

Each temperature cycle may have a duration of 20 seconds or below; or 15 seconds or below.

With such temperature cycle durations, it is possible to achieve PCR with the system according to the first aspect or embodiments.

Each temperature cycle may, for example, have a duration of 5-20 seconds, or 5-15 seconds.

Thus, it may be possible with the system to provide rapid nucleic acid analysis using PCR.

The light sensor may be configured to generate at least one signal, per temperature cycle over at least 5 number of temperature cycles, or 10 number of temperature cycles, or 45-55 number of temperature cycles.

Further, the light sensor may be configured to generate at least one signal, per temperature cycle over at least 1-100 number of temperature cycles, such as 5-60, 10-60, 15-60, 20-60, 25-60, 30-60, 40-60, 40-55, 41-54, 42-53, 43-52, 44-51, 45-50 number of temperature cycles.

Thereby, the PCR may be monitored over time and real-time PCR is achievable.

The first excitation wavelength range, the second excitation wavelength range, and the third excitation wavelength range may be different wavelength ranges.

Thereby, for example, a first, a second, and a third sequence or unique DNA may be monitored. Further, for example, three different viruses may be analysed, or three different type of fluorophores may be monitored.

The first emission wavelength range, the second emission wavelength range, and the third emission wavelength range are different wavelength ranges.

Thereby, for example, a first, a second, and a third sequence or unique DNA may be monitored. Further, for example, three different viruses may be analysed, or three different type of fluorophores may be monitored.

The first side and the second side of the sample portion may be parallel to a main side of the test card.

Thus, for example, a flat test card may be used.

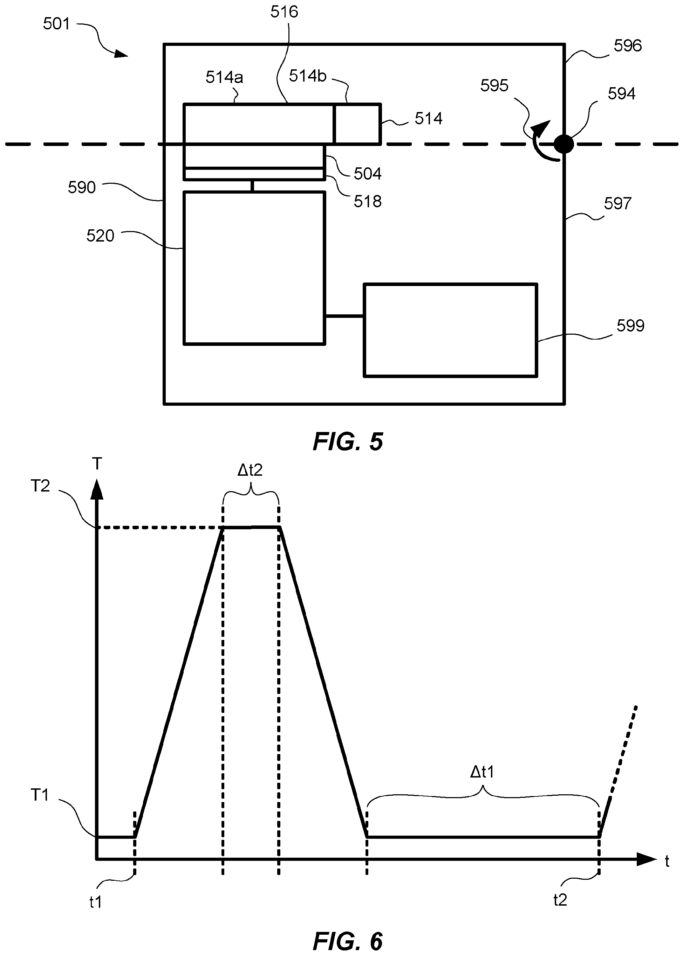

The system may be built into a housing, or the system may comprise a housing. With reference to figure 5, a system 500 comprising a housing 590 will now be discussed. The housing may comprise all details of the system, such as discussed with reference to figure 1, or as discussed with reference to other embodiments. Thereby, the housing may comprise, for example, a test card receiver 504 configured to receive the test card 6 (not illustrated). The first side 10 of the test card would be facing upwards in figure 5. The system 501 further comprises a temperature adjuster 514, for example comprising a peltier element 514a and a het sink 514b.The temperature adjuster 514 comprises a surface 516 configured to be in contact with the first side 10 of the sample portion 8 of the test card 6.

The test card receiver 504 comprises a spring member 518 configured to push the sample portion 8 of the test card 6 towards the surface 516 of the temperature adjuster 514. The system 1 further comprises a monitoring arrangement 520 as described earlier, such as, for example, with reference to figure 3. According to this example, the system is provided with an image sensor, and the collection optics is imaging optics configured to image the sample portion on the image sensor. The housing further comprises electronics 599 adapted to receive and transmit signals and/or control the heat adjuster 514, and the imaging sensor. The electronics 599 are further adapted to receive power and provide power within the system.

A hinge mechanism 594 allows a lid 596 of the house to be opened as indicated with arrow 595. The housing 590 in figure 5 is illustrated in a closed position. The spring member 518 provides force pushing the sample portion 8 of the test card 6 towards the surface 516 of the temperature adjuster 514. The Lid 596 further provides light tight enclosure to increase sensitivity of the system. The test card receiver 504 is arranged to receive a test card when the lid 596 of the housing is in an open position. The temperature adjuster 514 is provided in a fixed position in relation to the lid, while the test card receiver is provided in a fixed position in relation to a bottom portion 597 of the housing. Thereby, the system is arranged such that when the lid closes, extra force, in addition to force provided by the spring member 518, is provided by the surface 516 of the temperature adjuster pushing the sample portion 8 of the test card 6 towards the surface 516 of the temperature adjuster 514. In this example, the force from the spring member 518 and from surface 516 with the lid 596 in its closed position is approximately 9-11 N, or around 10 N. This force has been found suitable for a test card 6 having a silica chip comprising the sample portion 8. Desirable thermal contact is achieved allowing for rapid temperature cycles and thus rapid PCR, while the test card was not damaged. Higher temperatures were allowed with the silica chip as compared to a chip made of plastic material.

Although not illustrated in figure 5, the system may further comprise a bar code reader used for identifying individual test cards, and/or a USB hub used for transfer of information from the system.

With further reference to the discussions above, the spring member, such as a leaf spring which was used for the example above referring to figure 5, may be mounted fixed in relation to the housing. The spring member or leaf spring provides at least part of the force when the test card is received by the system and positioned in contact with the leaf spring. The lid of the housing may be configured to provide additional force on the sample portion, as the lid is in a closed position. The surface of the temperature adjuster may be considered to be mounted fixed in relation to the lid of the housing.

Other standard and/or useful features added to the system may further comprise the following additional features of the test card receiver: features allowing introduction of the testcard into the system under a slight angle of <2 degrees up and side; features allowing rough and precise alignment over the width of the system with direct relation to pins in the monitoring arrangement; features for preventing the test card hitting the temperature adjuster while receiving the test card in the system

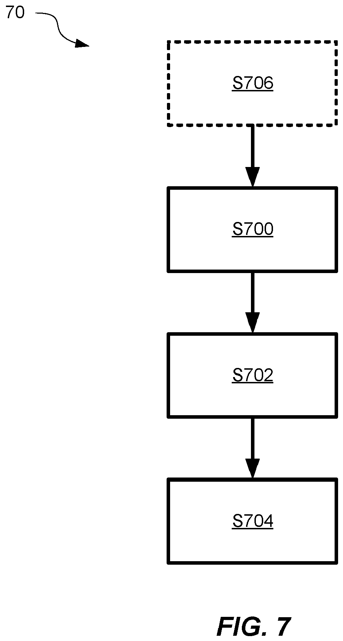

According to a second aspect there is provided a method 70 for performing PCR analysis using the system according to the first aspect. The method 70 is illustrated in figure 7. The method 70 comprising:
receiving S700 a test card at a test card receiver, wherein the test card comprises a sample portion having a first side and a second side opposite the first side, wherein the sample portion of the test card holds a sample for PCR,
adjusting S702 a temperature of the sample portion of the test card (6) over a plurality of temperature cycles, wherein each temperature cycle is between a first temperature and a second temperature different than the first temperature, using a temperature adjuster comprising a surface being in contact with the first side of the sample portion of the test card,
wherein the test card receiver comprises a spring member pushing the sample portion of the test card towards the surface of the temperature adjuster, and
monitoring S704 the sample portion of the test card over the plurality of temperature cycles, using a monitoring arrangement comprising:
   at least one light sensor,
   an illumination system illuminating the second side of the sample portion with light having wavelengths within at least three excitation wavelengths ranges,
   an emission filter transmitting light having wavelengths within at least three emission wavelength ranges, and
   collection optics collecting light emanating from the second side of the sample portion of the test card and transmitted through the emission filter, and wherein the collection optics further direct the collected light towards the light sensor wherin at least a portion of the collected light impinges on the light sensor,
   wherein the light sensor generates at least one signal, wherein each signal pertains to light impinging on the light sensor after a unique number of temperature cycles of the plurality of temperature cycles.

The method 70 may further comprise, prior to receiving S700 the test card, loading S706 the test card with sample for PCR.

According to a third aspect, there is provided a use of the system according to any one of claims the first aspect, for PCR analysis or nucleic acid analysis.

### EXAMPLE:

Nucleic acid analysis using PCR analysis was conducted using the system for imaging detection using three LEDs as light source. The system was provided in a housing as illustrated in figure 5. A first light source (red), and corresponding optics and filters, was dedicated towards one sequence of COVID-19. A second light source (green), and corresponding optics and filters, was dedicated towards another sequence of COVID-19. A third light source (orange), and corresponding optics and filters, was dedicated towards a reference sample. First, second and third, each different, fluorophores were provided for each sequence. Imaging optics and sensors were used for real time imaging. Sample was added to a silica chip of a test card.

Fifty heating cycles were run, each lasting for about ten seconds, adding up to a total analysis time of about eight minutes. Temperatures of the cycles were set in accordance with embodiments herein, and as illustrated in figure 6, illustrating the temperature cycle of the example. T is temperature and t is time. Although the rate of temperature increase is similar to the rate of temperature decrease in the example, other profiles of the temperature cycle may be realised and used. Thus, the rate of temperature increase may equal the rate of temperature decrease, or it may be larger or smaller. For this example, the temperature increase occurs during about two seconds and the temperature decrease occurs during about two seconds. For this example, the highest temperature, T2, was maintained for about a second, Δt2, and the lowest temperature, T1, was maintained for about five seconds, Δt1. In the example, T2 is about 100°C, T1 about 63°C, t1 is 0 s and t2 is about 10 s. An image was captured for each of the cycles. Resulting from the optics, an image associated with each of the light sources was imaged on a respective portion of the image sensor.

Presence of COVID-19 in the sample could successfully be detected and quantified in spite of the short total analysis time. This is believed to be, at least in part, due to the relatively high temperatures used, allowed with the combination of the material of the chip and the applied force between the chip and the surface of the temperature adjuster. The higher temperature was for the present example held a shorter time than the lower temperature was. This was beneficial and sped up the cycling time, and could be realised at least in part resulting from more rapid denaturing as compared to the annealing.

## Claims

1. A system (1) for PCR analysis, the system (1) comprising:
a test card receiver (4) configured to receive a test card (6), wherein the test card (6) comprises a sample portion (8) having a first side (10) and a second side (12) opposite the first side (10), wherein the sample portion (8) of the test card (6) is configured to hold a sample for PCR,
a temperature adjuster (14) configured to adjust a temperature of the sample portion (8) of the test card (6) over a plurality of temperature cycles, wherein each temperature cycle is between a first temperature and a second temperature different than the first temperature, wherein the temperature adjuster (14) comprises a surface (16) configured to be in contact with the first side (10) of the sample portion (8) of the test card (6),
wherein the test card receiver (4) comprises a spring member (18) configured to push the sample portion (8) of the test card (6) towards the surface (16) of the temperature adjuster (14), and
a monitoring arrangement (20) configured to monitor the sample portion (8) of the test card (6) over the plurality of temperature cycles, the monitoring arrangement (20) comprising:
at least one light sensor (22),
an illumination system (24) configured to illuminate the second side (12) of the sample portion (8) with light having wavelengths within at least three excitation wavelengths ranges,
an emission filter (26) configured to transmit light having wavelengths within at least three emission wavelength ranges, and
collection optics (28) configured to collect light emanating from the second side (12) of the sample portion (8) of the test card (6) and transmitted through the emission filter (26), and wherein the collection optics (28) is further configured to direct the collected light towards the light sensor (22) such that at least a portion of the collected light impinges on the light sensor (22),
wherein the light sensor (22) is configured to generate at least one signal (S), wherein each signal (S) pertains to light impinging on the light sensor (22) after a unique number of temperature cycles of the plurality of temperature cycles.

2. The system for PCR analysis according to claim 1, wherein the at least one light sensor is at least one image sensor, and the collection optics is imaging optics configured to image the sample portion on the image sensor.

3. The system according to claim 1, wherein the monitoring arrangement comprises:
the at least one light sensor
a first light source configured to illuminate the second side of the sample portion through a first excitation light filter, thereby providing light having wavelengths within a first excitation wavelength range at the second side of the sample portion of the testcard,
a first emission filter configured to transmit light having wavelengths within a first emission wavelength range,
a first collection optics configured to collect light emanating from the second side of the sample portion of the test card and transmitted through the first emission filter, and wherein the first collection optics is further configured to direct the collected light towards the light sensor such that at least a portion of the collected light impinges on the light sensor,
a second light source configured to illuminate the second side of the sample portion through a second excitation light filter, thereby providing light having wavelengths within a second excitation wavelength range at the second side of the sample portion of the testcard,
a second emission filter configured to transmit light having wavelengths within a second emission wavelength range,
a second collection optics configured to collect light emanating from the second side of the sample portion of the test card and transmitted through the second emission filter, and wherein the second collection optics is further configured to direct the collected light towards the light sensor such that at least a portion of the collected light impinges on the light sensor,
a third light source configured to illuminate the second side of the sample portion through a third excitation light filter, thereby providing light having wavelengths within a third excitation wavelength range at the second side of the sample portion of the testcard,
a third emission filter configured to transmit light having wavelengths within a third emission wavelength range, and
a third collection optics configured to collect light emanating from the second side of the sample portion of the test card and transmitted through the third emission filter, and wherein the third collection optics is further configured to direct the collected light towards the light sensor such that at least a portion of the collected light impinges on the light sensor.

4. The system according to claim 3, wherein
the at least one light sensor is at least one imaging sensor, and
each of the collection optics is a respective imaging optics configured to collect light transmitted through the emission filter and to image the second side of the sample portion of the test card on the least one image sensor.

5. The system according to claim 4, wherein the at least one image sensor is configured to capture at least one image of the second side of the sample portion, wherein each image is captured after a unique number of temperature cycles of the plurality of temperature cycles.

6. The system according to claim 3, wherein the at least one light sensor is at least one photodiode.

7. The system according to any one of claims 2 to 6, wherein each of the first light source, the second light source, and the third light source is a LED.

8. The system according to any one of the previous claims, wherein the spring member is configured to push the sample portion of the test card towards the surface of the temperature adjuster at a force of 5-15 N, such as 8-12 N.

9. The system according to any one of the previous claims, wherein the spring member comprises a leaf spring configured to allow transmitting light to and from the second side of the sample portion by having a slit or aperture.

10. The system according to any one of the previous claims, wherein the temperature adjuster comprising a thermoelectric element, such as a Peltier element.

11. The system according to any one of the previous claims, wherein one of the first temperature and the second temperature is within the interval 60-70°C and the other of the first temperature and the second temperature is within the interval 90-105°C.

12. The system according to any one of the previous claims, wherein the temperature adjuster is configured to provide 1 to 100 number of temperature cycles, such as 5-60, or 45-50 number of temperature cycles.

13. The system according to any one of the previous claims, wherein each temperature cycle has a duration of 20 seconds or below; or 15 seconds or below.

14. The system according to any one of the previous claims, wherein the light sensor is configured to generate at least one signal, per temperature cycle over at least 5 number of temperature cycles, or 10 number of temperature cycles, or 45-55 number of temperature cycles.

15. The system according to any one of claims 3 to 14, wherein the first excitation wavelength range, the second excitation wavelength range, and the third excitation wavelength range are different wavelength ranges.

16. The system according to any one of claims 3 to 14, wherein the first emission wavelength range, the second emission wavelength range, and the third emission wavelength range are different wavelength ranges.

17. The system according to any one of the previous claims, wherein the first side and the second side of the sample portion are parallel to a main side of the test card.

18. A method (70) for performing PCR analysis using the system according to any one of the claims 1 to 17, the method (70) comprising:
receiving (S700) a test card at the test card receiver (4), wherein the test card (6) comprises a sample portion (8) having a first side (10) and a second side (12) opposite the first side (10), wherein the sample portion (8) of the test card (6) holds a sample for PCR,
adjusting (S702) a temperature of the sample portion (8) of the test card (6) over a plurality of temperature cycles, wherein each temperature cycle is between a first temperature and a second temperature different than the first temperature, using a temperature adjuster (14) comprising a surface (16) being in contact with the first side (10) of the sample portion (8) of the test card (6),
wherein the test card receiver (4) comprises a spring member (18) pushing the sample portion (8) of the test card (6) towards the surface (16) of the temperature adjuster (14), and
monitoring (S704) the sample portion (8) of the test card (6) over the plurality of temperature cycles, using a monitoring arrangement (20) comprising:
at least one light sensor (22),
an illumination system (24) illuminating the second side (12) of the sample portion (8) with light having wavelengths within at least three excitation wavelengths ranges,
an emission filter (26) transmitting light having wavelengths within at least three emission wavelength ranges, and
collection optics (28) collecting light emanating from the second side (12) of the sample portion (8) of the test card (6) and transmitted through the emission filter (26), and wherein the collection optics (28) further direct the collected light towards the light sensor (22) wherin at least a portion of the collected light impinges on the light sensor (22),
wherein the light sensor (22) generates at least one signal (S), wherein each signal (S) pertains to light impinging on the light sensor (22) after a unique number of temperature cycles of the plurality of temperature cycles.

19. A use of the system according to any one of claims 1 to 17, for PCR analysis or nucleic acid analysis.
